# EUROPÄISCHE PATENTANMELDUNG

(11) **EP 2 135 640 A1**
(43) Veröffentlichungstag der Anmeldung: **23.12.2009**
(21) Anmeldenummer: 09159868.0
(22) Anmeldetag: 11.05.2009
(51) Int. Cl.: A61N 1/362, A61N 1/39

(54) **Elektrotherapieeinrichtung zur Behandlung tachykarder Rhythmusstörungen eines Herzens**

(30) Priorität: 10.06.2008 DE 102008002331
(71) Anmelder: Biotronik CRM Patent AG, 6341 Baar (CH)
(72) Erfinder: Dörr, Thomas, 12437, Berlin (DE)
(74) Vertreter: Lindner-Vogt, Karin L.

(57) **Zusammenfassung**

Die Erfindung betrifft ein Elektrotherapiesystem, insbesondere einen implantierbaren Herzstimulator, das als elektronisches Implantat zur elektrischen antitachykarden Therapie des Herzens mittels mindestens einer programmierbaren Therapiesequenz (d. h. einer Abfolge von mehreren Therapien, die nacheinander zur Behandlung einer VT/VF-Episode abgegeben werden) ausgebildet ist und einen Therapieerfolgsspeicher zur Abspeicherung einer Therapieerfolgsstatistik für jede Therapie aufweist, sowie eine Therapiesteuereinheit, die ausgebildet ist, eine Anpassung der Reihenfolge der Therapien innerhalb einer jeweiligen Therapiesequenz, automatisch abhängig von einer aktuell gespeicherten Therapieerfolgsstatistik, vorzunehmen.

## Beschreibung

Die Erfindung betrifft eine Elektrotherapieeinrichtung zur Behandlung tachykarder Rhythmusstörungen eines Herzens mit Hilfe von elektrischen Stimulationsimpulsen oder elektrischen Defibrillationsschocks.

Derartige Elektrotherapieeinrichtungen sind beispielsweise in Form implantierbarer Cardioverter/Defibrillatoren (ICDs) grundsätzlich bekannt und dienen dazu, solche tachykarden Rhythmusstörungen, wie Vorhofflattern oder Vorhofflimmern oder Kammerflattern oder Kammerflimmern, zu detektieren und durch gezielte Abgabe einer Therapiesequenz, die wenigstens einen Elektrostimulationsimpuls oder Defibrillationsschock enthält, möglichst zu beenden. Im Vordergrund stehen dabei die ventrikulären tachykarden Rhythmusstörungen, wie Kammerflattern (ventrikuläre Tachykardie) oder Kammerflimmern (ventrikuläre Fibrillation). Diese ventrikulären tachykarden Rhythmusstörungen können ihren Ursprung im Ventrikel selbst haben und werden dann als ventrikuläre Tachykardie im engeren Sinne (VT) oder als ventrikuläre Fibrillation (VF) bezeichnet. Wenn die ventrikuläre tachykarde Rhythmusstörung des Ventrikels ihren Ursprung im zugehörigen Atrium des Herzens oder im Sinusknoten hat, werden die ventrikulären tachykarden Rhythmusstörungen als supraventrikuläre Tachykardie (SVT) bezeichnet.

Während es bei einer ventrikulären Fibrillation aufgrund kreisender Erregung des Myokards (Herzmuskelgewebes) zu einer unkoordinierten Kontraktion des betroffenen Ventrikels kommt, die dazu führt, dass der Ventrikel nicht mehr in der Lage ist, Blut zu fördern, ist im Falle des Kammerflatterns häufig noch ein Rest einer koordinierten Kontraktion der Herzkammer festzustellen. Kammerflimmern und Kammerflattern unterscheiden sich beispielsweise dadurch, dass die Frequenz der Erregung im Falle des Kammerflimmerns noch höher ist als im Falle des Kammerflatterns. Die Erfassung und Differenzierungen der verschiedenen tachykarden Herzrhythmusstörungen sind grundsätzlich und hinlänglich bekannt.

Ebenso bekannt sind verschiedene Therapiesequenzen zur Beendigung einer tachykarden Herzrhythmusstörung. Zu diesen zählen antitachykarde Stimulation (anti tachycardia pacing, ATP) in Form einer Overdrive-Stimulation, bei der Stimulationsimpulse mit einer gegenüber über der der vorliegenden, intrinsischen (tachykarden) Herzrate erhöhten Stimulationsrate abgegeben werden, die Abgabe von Kardioversionsschocks oder die Abgabe von Defibrillationsschocks, wobei erstere üblicherweise eine geringere Energie haben als letztere. Defibrillationsschocks sollen das gesamte Myokard einer betroffenen Herzkammer gleichzeitig refraktär und damit für eine Erregung temporär unempfänglich machen, um so einen kreisende Erregung des betroffenen Herzmuskels zu unterbrechen. Andere Therapiesequenzen wie z. B. die antitachykarde Stimulation (ATP) umfassen mehrere, weniger energiereiche Stimulationsimpulse zur Kardioversion der betroffenen Herzkammer. Die Therapiesequenzen unterscheiden sich also beispielsweise durch die Anzahl der abgegebenen Stimulationsimpulse bzw. Defibrillationsschocks sowie durch den Zeitpunkt, zu dem einzelne Stimulationsimpulse bzw. Defibrillationsschocks abgegeben werden bzw. zu welchen ein Stimulationsimpuls auf einen vorangegangenen Stimulationsimpuls zur Kardioversion folgt.

Soll eine Kardioversion einer ventrikulären Tachykardie (VT, Kammerflattern) auf dem Wege einer antitachykarden Stimulation (anti tachycardia pacing, ATP) in Form einer Overdrive-Stimulation als Therapiesequenz erfolgen, werden Stimulationsimpulse mit einer gegenüber über der der vorliegenden, intrinsischen (tachykarden) Herzrate erhöhten Overdrive-Stimulationsrate abgegeben Dies soll bewirken, dass durch einen Stimulationsimpuls, der vor der natürlichen (intrinsischen) Erregung der betroffenen Herzkammer erfolgt, ein für ventrikuläre Tachykardien (VT, Kammerflattern) typischer Wiedereintritts-Zyklus (Reentry-Zyklus) der Erregung des Myokards unterbrochen wird.

Aus dem Stand der Technik sind implantierbare Kardioverter/Defibrillatoren (ICDs) bekannt, die dazu ausgebildet sind, eine tachykarde Rhythmusstörung zunächst zu detektieren und zur Behandlung einer detektierten tachykarden Herzrhythmusstörung Therapiesequenzen unterschiedlichen Rangs abzugeben. Dabei wird zunächst eine Therapiesequenz mit niedrigem Rang abgegeben, die üblicherweise die Abgabe von Stimulationsimpulsen niedrigerer Energie beinhaltet und damit für den Patienten erträglicher ist. Sollte eine rangniedrigere Therapiesequenz nicht zu einer erfolgreichen Beendigung der tachykarden Herzrhythmusstörung führen, wird eine ranghöhere Therapiesequenz ausgelöst, wobei üblicherweise die ranghöchste Therapiesequenz die (schmerzhafte) Abgabe eines Defibrillationsschocks ist. Darüber hinaus sind ICDs bekannt, die dazu ausgebildet sind, ventrikuläre Tachykardien zu detektieren und jeweils einer von mehreren VT-Zonen zuzuordnen. Dabei umfasst eine jeweilige VT-Zone ventrikuläre Tachykardien innerhalb eines jeweils anderen Herzratenbereichs. Die Detektion einer ventrikulären Tachykardie in einer bestimmten VT-Zone veranlasst diese bekannten ICDs dazu, jeweils zunächst diejenige Therapiesequenz auszulösen, die zuletzt zu einer erfolgreichen Beendigung der ventrikulären Tachykardie in dieser VT-Zone geführt hat. Sollte diese Therapiesequenz keinen Therapieerfolg nach sich ziehen (d. h. die ventrikuläre tachykarde Herzrhythmusstörung nicht beenden), wird anschließend eine andere Therapiesequenz ausgelöst. Sollte diese erforderlich sein, wird die andere Therapiesequenz bei der Detektion der nächsten tachykarden Herzrhythmusstörung in dieser VT-Zone als erste Therapiesequenz angewandt.

Aus US 2007/0049974 sind ein Verfahren und ein System bekannt, wie Therapiesequenzen unterschiedlichen Rangs auf Basis einer Morphologieanalyse des die tachykarde Herzrhythmusstörung charakterisierenden Elektrokardiogramms ausgewählt werden.

Die bekannten Mechanismen zur Anpassung der Therapiesequenz beschränken sich also auf die Steigerung der Schockenergie (d. h. den Übergang von einer niedrigeren zu einer ranghöheren Therapiesequenz) in Reaktion auf eine tachykarde Herzrhythmusstörung, falls die erste Therapiesequenz erfolglos sein sollte oder auf die einmalige Abgabe einer zuvor erfolgreichen Therapiesequenz oder das Schwächen einer wiederholt erfolglosen Therapiesequenz.

Der Erfindung liegt die Aufgabe zugrunde, einen antitachykarden Herzstimulator zu schaffen, der Therapiesequenzen zur Behandlung von Tachykardien zwischen zwei Nachsorgeintervallen möglichst selbständig hinsichtlich der Therapieeffizienz optimiert.

Erfindungsgemäß wird diese Aufgabe durch ein Elektrotherapiesystem, insbesondere einen implantierbaren Herzstimulator gelöst, das als elektronisches Implantat zur elektrischen antitachykarden Therapie des Herzens mittels mindestens einer programmierbaren Therapiesequenz (d. h. einer Abfolge von mehreren Therapien, die nacheinander zur Behandlung einer VT/VF-Episode abgegeben werden) ausgebildet ist und einen Therapieerfolgsspeicher zur Abspeicherung einer Therapieerfolgsstatistik für jede Therapie aufweist, sowie eine Therapiesteuereinheit, die ausgebildet ist eine Anpassung der Reihenfolge der Therapien innerhalb einer jeweiligen Therapiesequenz automatisch abhängig von einer aktuell gespeicherten Therapieerfolgsstatistik vorzunehmen.

Die Therapieerfolgsstatistik ist dabei vorzugsweise in Form von Therapieerfolgszählern für eine jeweilige antitachykarde Therapie verwirklicht. Die Therapieerfolgszählerstände sind dabei in dem Therapieerfolgsspeicher gespeichert. Immer, wenn die Therapieerfolgserfassungseinheit nach einer gerade abgegebenen antitachykarden Therapie einen Therapieerfolg, d. h. z. B. ein Ende der behandelten Tachykardie, erfasst, wird der Therapieerfolgszählerstand für diese Therapie um 1 erhöht.

Für verschiede Arten von Tachykardien (langsam, schnell, Fibrillation; jeweils mit stabilem oder instabilem Rhythmus) können dabei jeweils eigene Therapieerfolgszähler vorgesehen sein. Vorzugsweise ist die Steuereinheit des Therapiesystems dann so ausgebildet, dass sie nach Erfassen und kategorisieren einer Tachykardie zunächst diejenige antitachykarde Therapie aus einer Therapiesequenz auslöst, die für die jeweils detektierte Tachykardie den höchsten Zählerstand aufweist.

Bestandteile eines erfindungsgemäßen Therapiesystems sind in einer etwas ausführlicheren Auflistung:
- wenigstens eine Stimulationseinheit, die mit einer Elektrodenleitung mit einer intrakardialen Stimulations- oder Defibrillationselektrode verbunden oder zu verbinden ist und die dazu ausgebildet ist, elektrische Stimulationsimpulse und/oder elektrische Defibrillationsschocks zu generieren und abzugeben,
- einer (Therapie-) Steuereinheit, die mit der Stimulationseinheit verbunden ist und ausgebildet ist, die Stimulationseinheit zur Abgabe verschiedener antitachykarder Therapien entsprechend gespeicherter Therapiesequenzen anzusteuern, wobei eine jeweilige antitachykarde Therapie jeweils wenigstens die Abgabe eines Stimulations- oder Defibrillationsimpulses einschließt und sich verschiedene antitachykarde Therapien einer Therapiesequenz durch wenigstens ein Therapiemerkmal, wie Stärke des Stimulationsimpulses oder Defibrillationsimpulses, Anzahl der Impulse oder Reihenfolge und Zeitpunkte einzelner Impulse, voneinander unterscheiden,
- einer Tachykardieerfassungseinheit, die über eine Elektrodenleitung mit einer intrakardialen Wahrnehmungselektrode verbunden oder zu verbinden ist und die dazu ausgebildet ist, ein intrakardiales Elektrokardiogrammsignal aufzunehmen und derart zu verarbeiten, dass die Tachykardieerfassungseinheit eine Tachykardie eines Vorhofs oder einer Kammer eines Herzens detektieren und eine jeweilige Tachykardie kennzeichnende Merkmale erfassen kann,
- einer Therapieerfolgserfassungseinheit, die mit einem Therapieerfolgsspeicher verbunden und ausgebildet ist, ein Ende einer tachykarden Rhythmusstörung nach Abgabe einer Therapiesequenz als Therapieerfolg zu erfassen und in dem Therapieerfolgsspeicher Daten zu speichern, die die tachykarde Rhythmusstörung charakterisieren sowie diejenige antitachykarde Therapie innerhalb einer jeweiligen Therapiesequenz, die zur erfolgreichen Beendigung der tachykarden Rhythmusstörungen geführt hat, und auf Basis der in dem Therapieerfolgsspeicher gespeicherte Daten eine Therapieerfolgsstatistik zu erstellen,
- wobei die Steuereinheit mit dem Therapieerfolgsspeicher verbunden und dazu ausgebildet ist, auf Basis der in dem Therapieerfolgsspeicher abgelegten Therapieerfolgsstatistik die Reihenfolge von antitachykarden Therapien einer zukünftigen, nach Erfassung einer tachykarden Rhythmusstörung anzuwendenden Therapiesequenz derart zu bestimmen, dass die Reihenfolge der antitachykarden Therapien innerhalb der Therapiesequenz davon abhängt, wie häufig eine antitachykarde Therapie im Vergleich zu anderen antitachykarden Therapien der Therapiesequenz zu einer erfolgreichen Beendigung einer Tachykardie geführt hat.

Die Therapieerfassungseinheit kann dabei Bestandteil der Steuereinheit sein und veranlasst, dass die Steuereinheit ggf. eine antitachykarde Therapie aus einer entsprechenden Therapiesequenz auslöst.

Wie zuvor bereits mit anderen Worten erwähnt ist die Therapieerfolgserfassungseinheit in Verbindung mit dem Therapieerfolgspeicher vorzugsweise als Therapieerfolgszähler dazu ausgebildet, einen gespeicherten Zählerstand zu einer jeweiligen antitachykarden Therapie zu erhöhen, wenn diese antitachykarde Therapie erfolgreich war. Die Zählerstände der Therapieerfolgszähler bilden dann die Therapieerfolgsstatistik.

Vorzugsweise ist die Tachykardieerfassungseinheit ausgebildet, stabile und instabile Tachykardien - d. h. Tachykardien, die mit einem stabilen bzw. instabilen (also unregelmäßigen) Herzrhythmus einhergehen - voneinander zu unterscheiden. Und die Therapieerfolgserfassungseinheit ist in Verbindung mit dem Therapieerfolgspeicher als Therapieerfolgszähler dazu ausgebildet, einen von zwei einer jeweiligen antitachykarden Therapie zugeordneten Zählerstände zu erhöhen, von denen einer instabilen Tachykardien zugeordnet ist und der andere stabilen Tachykardien, je nachdem, ob eine jeweils erfolgreich beendete Tachykardie von der Tachykardieerfassungseinheit als stabil oder als instabil klassifiziert wurde. Mit anderen Worten: Für eine jeweilige antitachykarde Therapie sind zwei Therapieerfolgszähler vorgesehen, nämlich einer, der ggf. nach erfolgreicher antitachykarder Therapie einer Tachykardie mit instabilem Rhythmus hochgezählt wird, und einer, der ggf. nach erfolgreicher antitachykarder Therapie einer Tachykardie mit stabilem Rhythmus hochgezählt wird. Auf diese Weise können die Therapiesequenzen für unterschiedliche Arten von Tachykardien spezifisch optimiert werden.

Entsprechend sind vorzugsweise für jede antitachykarde Therapie jeweils separate Therapieerfolgszähler für verschieden "schnelle" Tachykardien vorgesehen, also z. B. langsame Tachykardien, die mit einer Herzrate einhergehen, die in einen vorgegebenen Bereich von Herzraten fällt, der mit "VT-Zone 1" bezeichnet wird, schnelle Tachykardien, die in eine "VT2 Zone" fallen, die einem Bereich höherer Herzraten zugeordnet ist als die "VT1 Zone" und ventrikuläre Fibrillationen (VF), die in einen Bereich sehr hoher Herzraten fallen, der mit "VF Zone" bezeichnet wird.

Hierzu ist die Tachykardieerfassungseinheit vorzugsweise ausgebildet, eine erfasste Tachykardie einer von mindestens zwei Tachykardiezonen derart zuzuordnen, dass die Zuordnung zu einer der Tachykardiezonen in Abhängigkeit einer tachykarden Herzrate eindeutig erfolgt, wobei die Steuereinheit dazu ausgebildet ist, je nach Zuordnung einer detektierten Tachykardie eine von wenigstens zwei Therapiesequenzen, die jeweils einer Tachykardiezone zugeordnet sind, anzuwenden.

Weiter ist die Tachykardieerfassungseinheit vorzugsweise ausgebildet, eine sich beschleunigende Tachykardie nach Abgabe einer antitachykarden Therapie zu erfassen.

In Verbindung hiermit kann die Steuereinheit ausgebildet sein, nach Erfassen einer sich beschleunigenden Tachykardie infolge der Abgabe einer antitachykarden Therapie den zugehörigen Therapieerfolgszähler um 1 herabzuzählen oder dessen Zählerstand auf "0" zu setzen, um so zu bewirken, dass die antitachykarde Therapie in der jeweiligen Therapiesequenz allenfalls nachrangig abgegeben wird.

Alternativ hierzu kann der Therapieerfolgsspeicher ausgebildet sein, zu einer jeweiligen antitachykarden Therapie eine Sperrkennung zu speichern, und die Therapieerfolgserfassungseinheit ausgebildet sein, die Sperrkennung zu einer jeweiligen antitachykarden Therapie in den Therapieerfolgsspeichern, wenn diese antitachykarde Therapie zu einer sich beschleunigenden Tachykardie geführt hat. Die Steuereinheit ist dann ausgebildet, eine mit einer Sperrkennung gekennzeichnete antitachykarde Therapie aus einer jeweils neu gebildeten Therapiesequenz ganz auszunehmen, so dass eine mit einer Sperrkennung versehene antitachykarde Therapie zur Behandlung einer jeweiligen Tachykardie, zu deren Beschleunigung die antitachykarde Therapie geführt hat, nicht mehr abgegeben wird. Die jeweilige Sperrkennung ist somit jeweils therapie- und tachykardiespezifsch.

Vorzugsweise sind außerdem jeweils separate Therapieerfolgszähler für die grundlegende Therapieart antitachykarde Stimulation (ATP) und Schockabgabe vorgesehen. Die grundlegenden Arten antitachykarder Therapien können ihrerseits jeweils eine Reihe von Einzeltherapien repräsentieren. So können verschiedene Formen der Schockabgabe je nach Energie, Impulsform (z. B. monophasisch, biphasisch) und Polarität unterschieden und als verschiedene antitachykarde Therapien jeweils einem eigenen Therapieerfolgszähler zugeordnet sein.

Es ergeben sich somit folgende Varianten verschiedener, jeweils bevorzugter Gestaltungen des Therapieerfolgspeichers:
(a) Die Therapieerfolgszählerstände werden getrennt für ATP und Schock aufgezeichnet.
(b) Die Therapieerfolgszählerstände werden getrennt für jede einzelne Therapien aufgezeichnet.
(c) Die Therapieerfolgszählerstände werden getrennt für jede einzelne Therapie und jeweils getrennt für stabile und instabile Tachykardien aufgezeichnet.
(d) Die Therapieerfolgszählerstände für Schocks werden getrennt für jede Schockenergie aufgezeichnet.
(e) Die Therapieerfolgszählerstände für Schocks werden getrennt für jede Schockpolarität aufgezeichnet.
(f) Die Therapieerfolgszählerstände für Schocks werden getrennt für jede Schockimpulsform aufgezeichnet.

Auch kommen Kombinationen der Gestaltungen gemäß (c) bis (e) in Frage.

Hinsichtlich der Behandlung der Zählerstände sowie der Optimierung der jeweiligen Therapiesequenz ergeben sich folgende, jeweils vorteilhafte Varianten:
- Eine Akzeleration des Rhythmus, ausgelöst durch eine jeweilige antitachykarde Therapie, veranlasst die Steuereinheit dazu, die Erfolgsquote der jeweiligen Therapie in der Therapieerfolgsstatistik zu löschen oder zu reduzieren.
- Eine Akzeleration des Rhythmus, ausgelöst durch die Therapie, veranlasst die Steuereinheit dazu, die jeweilige ATP-Therapie in der Therapieerfolgsstatistik zu sperren.
- Die zur Therapiesequenzoptimierung verwendeten Statistiken können mittels eines externen Programmiergerätes (Programmer) bei der Nachsorge durch einen Arzt abgefragt werden.
- Die Therapieerfolgszählerstände in dem Therapieerfolgsspeicher können vom Arzt einzeln oder insgesamt mittels Programmer gelöscht werden.
- Die Therapieerfolgszählerstände in dem Therapieerfolgsspeicher werden bei der Umprogrammierung der Therapiesequenz automatisch angepasst (gelöscht oder korrigiert).
- Die Therapiesequenzoptimierung durch die Steuereinheit ist mittels eines programmierbaren Parameters vom Arzt ein- und ausschaltbar.

Gemäß einer besonders bevorzugten Ausführungsvariante ist die Elektrotherapieeinrichtung wie folgt ausgebildet:

Für jeden ATP-Angriff und jeden Schock in den VT-Zonen werden zwei Therapieerfolgszähler abgespeichert, Therapieerfolg bei stabiler VT und Therapieerfolg bei instabiler VT.

Für die Stabilitätsbewertung wird immer das ventrikuläre Stabilitätskriterium unmittelbar vor der jeweiligen Detektion bzw. Redetektion genutzt.

Für jeden Schock in der VF-Zone wird ein Therapieerfolgszähler abgespeichert.

Der Therapieerfolgszähler wird inkrementiert, wenn die Therapie zur Terminierungsdetektion der Episode führt.

Der Therapieerfolgszähler wird dekrementiert, wenn der Therapie eine Redetektion in der gleichen oder niedrigeren Zone folgt.

Der Therapieerfolgszähler wird gelöscht, wenn der Therapie eine Redetektion in einer höheren Zone (Akzeleration) folgt.

Der Therapieerfolgszähler bleibt unverändert, wenn die Therapie (DFc) oder die Episode (Programmer) abgebrochen wurde.

Die Therapieerfolgszähler werden nur von spontanen Episoden beeinflusst, d. h. nicht bei induzierten Episoden.

Die Therapieerfolgszähler können bei der Nachsorge vom Programmer abgefragt werden.

Die Therapieerfolgszähler können mit dem Programmer manuell zurückgesetzt werden.

Die Therapieerfolgszähler werden bei Umprogrammierung der Therapiesequenzen, ATP und Schockparameter automatisch durch den Programmer zurückgesetzt.

Wird ein Schock mit einer Energie kleiner Maximalenergie von einer Redetektion gefolgt, dann wird im Therapieerfolgszähler ein Flag "Energieerhöhung" gesetzt, der Therapieerfolgszähler wird in diesem Fall nicht dekrementiert.

Wenn "Prefer ATP" ausgeschaltet ist, dann werden ATPs, die zu einer Akzeleration führen, gesperrt (spez. Code im Therapieerfolgszähler).

Wenn die Automatische Therapieoptimierung eingeschaltet ist, dann wird die Reihenfolge der Therapiesequenz entsprechend des Zählerstandes der Therapieerfolgszähler umsortiert:

Zunächst wird die programmierte Anzahl der ATP-Versuche abgegeben, jedoch nicht in der programmierten Reihenfolge, sondern in der Reihenfolge absteigenden Therapieerfolges, getrennt nach stabiler und instabiler VT zum Detektions-/ Redetektionszeitpunkt.

Sind alle ATP-Versuche erfolglos abgegeben worden, dann werden die Anzahl programmierter Schocks in der Reihenfolge absteigenden Therapieerfolges abgegeben. Ist die Energieerhöhung bei einem Schock markiert, wird die Schockenergie dieses Schocks auf Maximalenergie gesetzt.

Weitere bevorzugte Ausführungsvarianten ergeben sich aus nicht explizit erwähnten Kombinationen der hier beschriebenen Merkmale.

Die Erfindung soll nun anhand eines Ausführungsbeispiels mit Bezug auf die Figuren näher erläutert werden. Von den Figuren zeigt:
- Fig. 1:: einen implantierbaren Dreikammer-Kardioverter/Defibrillator als beispielhaften Herzstimulator in Verbindung mit daran angeschlossenen Elektrodenleitungen;
- Fig. 2:: ein schematisches Blockschaltbild des Herzstimulators aus Figur 1;
- Fig. 3:: eine Therapiesequenzstruktur, wie sie in derzeitigen ICD-Systemen verfügbar ist;
- Fig. 4:: die Struktur eines Therapieerfolgsspeichers;
- Fig. 5:: eine Speicherstruktur der Therapieerfolgszähler;
- Fig. 6:: eine Illustration der Zählweise des ATP-Therapieerfolgszählers;
- Fig. 7:: eine Illustration der Zählweise des Schock-Therapieerfolgszählers;
- Fig. 8:: die Anpassung der Therapiesequenz entsprechend Therapieerfolgszähler; und
- Fig. 9:: eine beispielhafte Übersicht über verschiedene Therapieparameter für einen Defibrillationsschock.

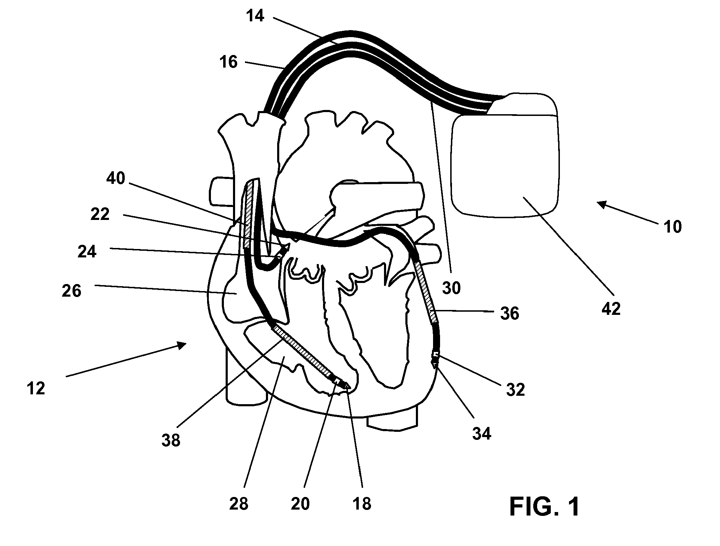

Fig. 1 zeigt ein Implantat 10 in Form eines biventrikulären Dreikammer-Herzschrittmachers und Cardioverters/Defibrillators (ICD). An diesen sind drei Elektrodenleitungen angeschlossen, nämlich eine rechtsatriale Elektrodenleitung 14, eine rechtsventrikuläre Elektrodenleitung 16 und eine linksventrikuläre Elektrodenleitung 30. Im implantierten Zustand endet die rechtsatriale Elektrodenleitung 14 im rechten Atrium 26 eines Herzens 12. Die rechtsventrikuläre Elektrodenleitung 16 endet im rechten Ventrikel 28 des Herzens 12 und die linksventrikuläre Elektrodenleitung 30 reicht über den Coronarsinus des Herzens 12 bis zum linken Ventrikel des Herzens.

Die rechtsatriale Elektrodenleitung 14 trägt an ihrem distalen Ende eine rechtsatriale Spitzenelektrode RA Tip 22 und in geringem Abstand davon eine rechtsatriale Ringelektrode RA Ring 24. In ähnlicher Weise trägt die rechtsventrikuläre Elektrodenleitung 16 an ihrem distalen Ende eine rechtsventrikuläre Spitzenelektrode RV Tip 18 und wenig entfernt davon eine rechtsventrikuläre Ringelektrode RV Ring 20. Auch am distalen Ende der linksventrikulären Elektrodenleitung 30 ist eine linksventrikuläre Spitzenelektrode LV Tip 34 und wenig entfernt davon eine linksventrikuläre Ringelektrode LV Ring 32 angebracht. Diese Elektroden dienen der Aufnahme elektrischer Potenziale in der jeweiligen Herzkammer sowie der Abgabe von Stimulationsimpulsen an die jeweilige Herzkammer im normalen Schrittmacherbetrieb. Dies braucht an dieser Stelle nicht mehr erläutert zu werden.

Die rechtsventrikuläre Elektrodenleitung 16 trägt außerdem noch eine im implantierten Zustand im rechten Ventrikel angeordnete rechtsventrikuläre Schockwendel RV Coil 38 als Defibrillationselektrode sowie eine im implantierten Zustand in der Vena cava superior befindliche zweite Schockwendel SVC Coil 40. Auch an der linksventrikulären Elektrodenleitung 30 ist eine linksventrikuläre Schockwendel LV Coil 36 angebracht. Die Schockwendeln dienen im Bedarfsfall als Defibrillationselektroden zur Abgabe von Defibrillationsschocks. Auch dies braucht an dieser Stelle nicht näher erläutert zu werden.

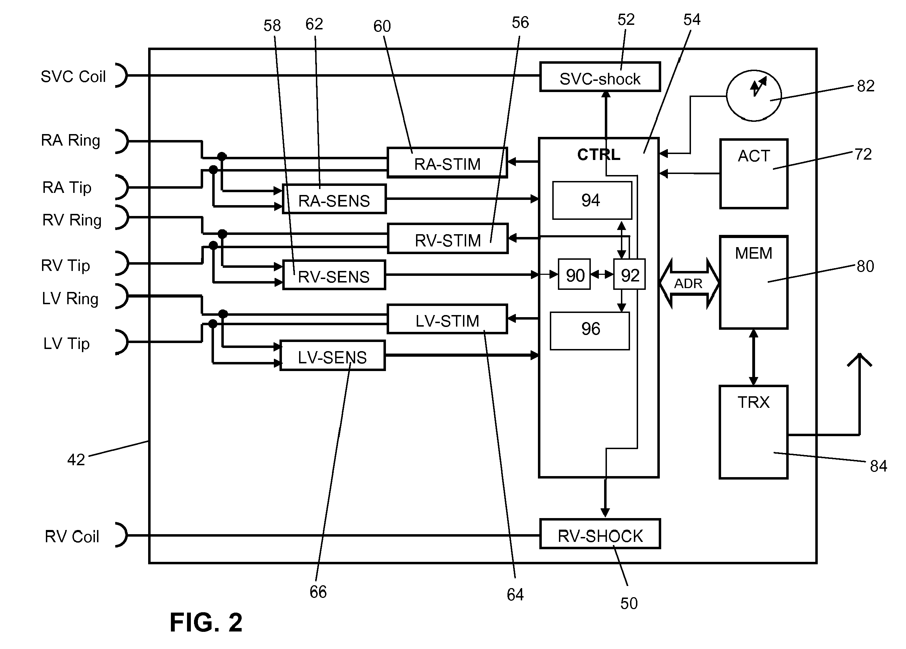

In Figur 2 sind die Hauptbestandteile des Herzstimulators 10 dargestellt. Auf der linken Seite sind die elektrischen Anschlüsse für die verschiedenen Elektroden 18, 20, 24, 22, 26, 32, 34, 38 und 40 dargestellt. Die Schockelektroden RV Coil 38 und SVC Coil 40 sind jeweils mit einem rechtsventrikulären Schockimpulsgenerator 50 bzw. SVC-Schockgenerator 52 verbunden. Beide Schockgeneratoren 50 und 52 sind mit einer Stimulationssteuereinheit 54 verbunden, die die beiden Schockimpulsgeneratoren 50 und 52 bei Bedarf zur Erzeugung und Abgabe eines Defibrillationsschocks ansteuert. Gegebenenfalls kann auch die Schockelektrode LV Coil 36 mit einem (in Figur 2 nicht dargestellten) Schockgenerator verbunden sein, der ebenfalls durch die Stimulationssteuereinheit 54 angesteuert wird.

Der Anschluss für die rechtsventrikuläre Spitzenelektrode RV Tip 18 sowie der Anschluss für die rechtsventrikuläre Ringelektrode RV Ring 20 sind jeweils sowohl mit einer rechtsventrikulären Stimulationseinheit 56 als auch mit einer rechtsventrikulären Sensingeinheit 58 verbunden. Sowohl die rechtsventrikuläre Stimulationseinheit 56 als auch die rechtsventrikuläre Sensingeinheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und im Anschluss für die rechtsventrikuläre Ringelektrode RV Ring 20 und die rechtsventrikuläre Spitzenelektrode RV Tip 18 abzugeben. Alternativ ist es auch möglich, dass das Gehäuse 42 des Herzstimulators 10 eine neutrale Elektrode bildet und die rechtsventrikuläre Stimulationseinheit 56 mit dem Anschluss für die rechtsventrikuläre Spitzenelektrode RV Tip 18 und dem Gehäuse 42 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode RV Tip 18. Diese Erregung breitet sich dann durch natürliche Reizleitung über den gesamten rechten Ventrikel 28 weiter aus und sorgt so für eine stimulierte Kontraktion des rechten Ventrikels 28.

Die rechtsventrikuläre Sensingeinheit 58 ist dazu ausgebildet, an dem Anschluss für die rechtsventrikuläre Ringelektrode RV Ring und die rechtsventrikuläre Spitzenelektrode RV Tip anliegende elektrische Potentiale zunächst durch einen Eingangsverstärker zu verstärken und zu filtern. Weiterhin ist die rechtsventrikuläre Sensingeinheit 58 ausgebildet, den Verlauf der an ihren Eingängen anliegenden elektrischen Signale derart auszuwerten, dass die rechtsventrikuläre Sensingeinheit 58 selbsttätig eine natürliche (intrinsische), d. h. selbsttätige Kontraktion des rechten Ventrikels 28 detektiert. Dies kann beispielsweise dadurch geschehen, dass der Verlauf des an den Eingängen der rechtsventrikulären Sensingeinheit 58 anliegenden Signals mit einem Schwellwert verglichen wird. Typischerweise ist die größte Amplitude des Signals in Form der sogenannten R-Zacke kennzeichnend für eine natürliche Kontraktion des rechten Ventrikels 28, die durch Schwellwertvergleich detektiert werden kann. Die rechtsventrikuläre Sensingeinheit 58 gibt daraufhin ein entsprechendes, eine natürliche Kontraktion des rechten Ventrikels 28 anzeigendes Ausgangssignal an die Stimulationssteuereinheit 54 aus.

In analoger Weise sind der Anschluss für die rechtsatriale Spitzenelektrode RA Tip 22 und der Anschluss für die rechtsatriale Ringelektrode RA Ring 24 sowohl mit einer rechtsatrialen Stimulationseinheit 60 als auch mit einer rechtsatrialen Sensingeinheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen als die rechtsventrikulären Stimulationsimpulse. Die rechtsatriale Sensingeinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums 26 kennzeichnet. Detektiert die rechtsatriale Sensingeinheit 62 eine entsprechende P-Welle, erzeugt sie ein Ausgangssignal und gibt dieses an die Stimulationssteuereinheit 54 weiter, welches eine natürliche Kontraktion des rechten Atriums 26 kennzeichnet.

In gleicher Weise sind auch der Anschluss für die linksventrikuläre Spitzenelektrode LV Tip 34 und der Anschluss für die linksventrikuläre Ringelektrode LV Ring 32 mit einer linksventrikulären Stimulationseinheit 64 und einer linksventrikulären Sensingeinheit 66 verbunden. Die linksventrikuläre Stimulationseinheit 64 und die linksventrikuläre Sensingeinheit 66 sind ebenso mit der Stimulationssteuereinheit 54 verbunden. Beide funktionieren ähnlich wie die bereits beschriebenen Stimulationseinheiten 56 und 60 und Sensingeinheiten 58 und 62.

Als weiterer Bestandteil des Herzstimulators 10 ist ein Aktivitätssensor 72 mit der Stimulationssteuereinheit 54 verbunden und in das Gehäuse 42 des Herzstimulators 10 integriert. Der Aktivitätssensor 72 ist dazu ausgebildet, ein von der körperlichen Aktivität eines Patienten abhängiges Bewegungssignal zu erfassen und ein entsprechendes, die körperliche Aktivität des Patienten anzeigendes erstes Signal an die Stimulationssteuereinheit 54 auszugeben. Dies erlaubt es, dass die Stimulationssteuereinheit 54 das Timing der Stimulationsimpulse an den Bedarf des Patienten (hämodynamischen Bedarf) anpasst.

Weiterhin umfasst der Herzstimulator 10 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern. Des Weiteren ist die Stimulationssteuereinheit 54 mit einem Zeitgeber 82 verbunden.

Die Speichereinheit 80 ist mit einer Telemetrieeinheit 84 verbunden, die es erlaubt, in der Speichereinheit 80 abgelegte Daten drahtlos an das externe Gerät 100 zu übertragen oder Programmierbefehle seitens des externen Geräts 100 zu dem Herzstimulator 10 zu übertragen und in der Speichereinheit 80 zu speichern.

Die Steuereinheit 54 ist dazu ausgebildet, verschiedene Therapiesequenzen zur Behandlung tachykarder Rhythmusstörungen auszulösen und zu steuern. Hierzu ist die Steuereinheit insbesondere ausgebildet, detektierte ventrikuläre Tachykardien in jeweils von drei Tachykardiezonen (ventrikuläre Tachykardie niederer Ventrikelfrequenz, VT1, ventrikuläre Tachykardie höherer Ventrikelfrequenz, VT2 und ventrikuläre Fibrillation, VF) zu klassifizieren, je nachdem, wie hoch die detektierte ventrikuläre Herzrate (Ventrikelfrequenz) ist.

Die Steuereinheit 54 besitzt im Sinne der Erfindung verschiedene Untereinheiten, nämlich eine Tachykardieerfassungseinheit 90, die dazu ausgebildet ist, anhand der von der rechtsventrikulären Sensingeinheit 58 gelieferten Senseereignisse eine Tachykardie zu detektieren und zu klassifizieren, nämlich je nach Herzrate in eine Tachykardie der VT-Zone 1, der VT-Zone 2 oder der VF-Zone. Daneben ist eine eigentliche Therapiesteuereinheit 92 vorgesehen, die dazu ausgebildet ist, nach Erfassen einer Tachykardie durch die Tachykardieerfassungseinheit 90 den rechtsventrikulären und/oder den linksventrikulären Schockgenerator 50 bzw. 52 oder die rechtsventrikuläre Stimulationseinheit 56 anzusteuern, um eine detektierte ventrikuläre Tachykardie entweder durch Abgabe einer antitachykarden Stimulation (ATP) über die rechtsventrikuläre Stimulationseinheit 56 oder durch Abgabe eines Defibrillationsschocks über die rechtsventrikuläre Schockwendel 38 oder die Schockwendel 40 in der Vena cava superior zu behandeln. In Verbindung mit der Tachykardieerfassungseinheit 90 bildet die Therapiesteuereinheit 92 auch eine Therapieerfolgserfassungseinheit, die nach der Abgabe einer jeweiligen antitachykarden Therapie (also einer antitachykarden Stimulation oder eines Defibrillationsschocks) durch Auswertung der ventrikulären Herzrate (anhand der von der rechten Sensingeinheit 58 gelieferten Senseereignisse) ermittelt, ob die Behandlung der Tachykardie erfolgreich war und somit keine Tachykardie mehr vorliegt oder ob die Behandlung der Tachykardie nicht erfolgreich war und die Tachykardie fortbesteht. Weiterhin ist die Therapiesteuereinheit 92 in Verbindung mit der Tachykardieerfassungseinheit 90 dazu ausgebildet, gegebenenfalls auch eine sich beschleunigende Tachykardie infolge der Abgabe einer antitachykarden Stimulation oder der Abgabe eines Defibrillationsschocks zu detektieren.

Welche antitachykarde Therapie (welche Art von antitachykarder Stimulation oder welcher Art von Defibrillationsschock) die Therapiesteuereinheit 92 in Reaktion auf eine detektierte Tachykardie abgibt, hängt zum einen von der Art der detektierten Tachykardie ab, nämlich in welche Zone die Tachykardie fällt und ob die Tachykardie stabil oder instabil ist, und zum anderen von einer in einem Therapiesequenzspeicher 94 gespeicherten Therapiesequenz. Dabei ist jeder Art von Tachykardie jeweils eine eigene Therapiesequenz zugeordnet, also eine Therapiesequenz für eine Tachykardie der VT-Zone 1, eine Therapiesequenz für eine Tachykardie der VT-Zone 2 und eine Therapiesequenz für eine ventrikuläre Fibrillation (VF-Zone). Eine jeweilige Therapiesequenz enthält verschiedene antitachykarde Therapien in einer gespeicherten Reihenfolge. Die verschiedenen antitachykarden Therapien können einmal danach unterschieden werden, ob es sich um antitachykarde Stimulationen (ATP) oder Defibrillationsschocks handelt. Die antitachykarden Stimulationen können wieder je nach Energie der Stimulationsimpulse und dem Zeitpunkt der Abgabe der Stimulationsimpulse unterschieden werden. Die Defibrillationsschocks können ebenfalls nach Schockenergie, Schockpolarität und Impulsform (beispielsweise monophasisch oder biphasisch) unterschieden werden. Entsprechend gibt es mehrere verschiedene antitachykarde Stimulationen und mehrere verschiedene Defibrillationsschocks innerhalb einer Therapiesequenz.

Zur ständigen Optimierung der Therapiesequenz durch die Therapiesteuereinheit 92 selbst ist ein Therapieerfolgsspeicher 96 vorgesehen, der eine Therapieerfolgsstatistik enthält. Diese Therapieerfolgsstatistik wird von verschiedenen Zählerständen gebildet, wobei jeder Zählerstand einer jeweiligen antitachykarden Therapie und einer jeweils zu behandelnden Tachykardie zugeordnet ist, wie dies nachfolgend noch näher erläutert wird. Die Therapiesteuereinheit 92 ist dazu ausgebildet, die einzelnen Zählerstände der Therapieerfolgsstatistik in dem Therapieerfolgsspeicher 96 zu erhöhen oder zu erniedrigen, je nachdem, ob eine jeweils gerade abgegebene antitachykarde Therapie zu einem Therapieerfolg oder zu keinem Therapieerfolg oder gar zu einer Beschleunigung der zu behandelnden Tachykardie geführt hat. Weiterhin ist die Therapiesteuereinheit 92 dazu ausgebildet, die in dem Therapiesequenzspeicher 94 gespeicherten Therapiesequenzen anhand der Therapieerfolgszählerstände in dem Therapieerfolgsspeicher 96 jeweils neu so zu arrangieren, dass innerhalb einer jeweiligen Therapiesequenz diejenige antitachykarde Therapie zuerst abgegeben wird, deren Therapieerfolgszählerstand der höchste ist und die somit die größten Erfolgsaussichten zur Behandlung der jeweils spezifischen detektieren Tachykardie verspricht.

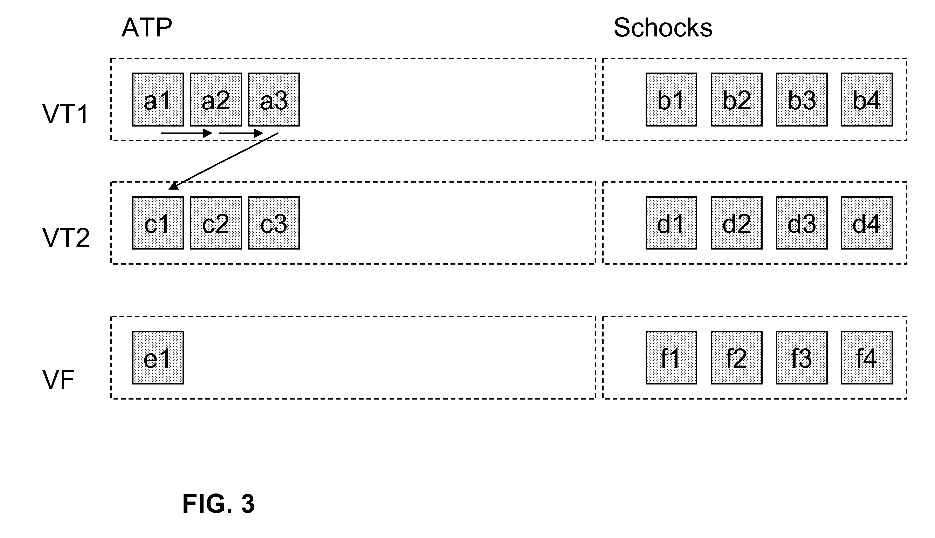

In Figur 3 ist eine Therapiesequenzstruktur dargestellt, wie sie in derzeitigen ICD-Systemen verfügbar ist. Je Zone (VT1, VT2, VF) ist eine Anzahl antitachykarder Therapieversuche zugeordnet. Zunächst werden in ATP-Therapien definiert und anschließend mehrere Defibrillationsschocks. Diese Therapieversuche können mit dem Programmiergerät mittels mehrerer Parameter, wie z. B. Anzahl der Stimulationsimpulse und Stimulationsintervall für ATP, Schockenergie, Schockpolarität oder Schockimpulsform, individuell konfiguriert werden.

Findet eine initiale Detektion in einer der VT/VF-Zonen statt, dann wird die Therapiesequenz jedes Mal in derselben Reihenfolge von links nach rechts abgegeben, bis entweder ein Therapieerfolg (Terminierungsdetektion) registriert oder alle Therapien der Therapiesequenz aufgebraucht sind.

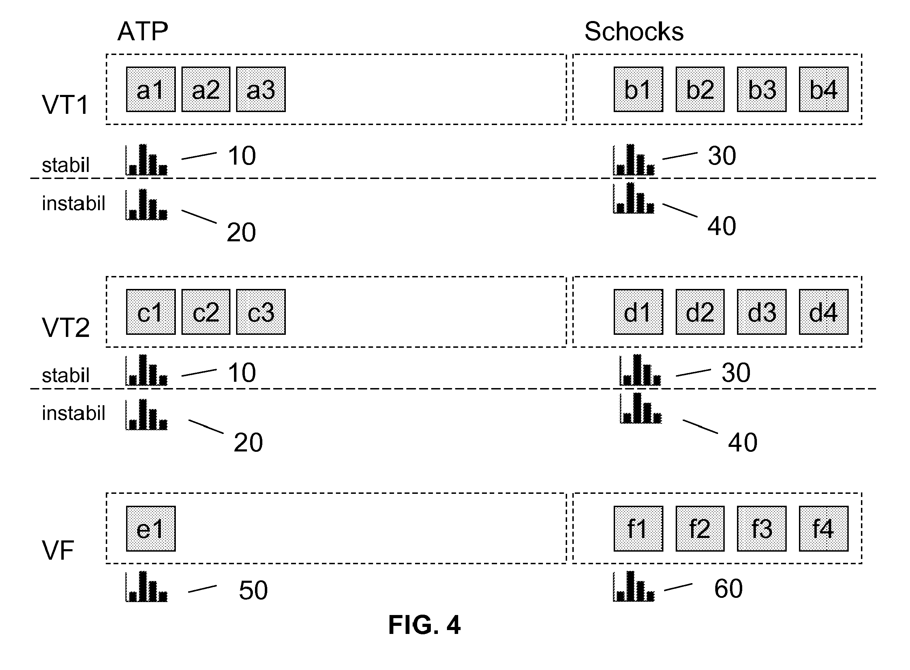

In Figur 4 ist die erfindungsgemäße Erweiterung einer Therapiesequenzstruktur dargestellt. Hier wird ein zusätzlicher Speicherbereich im elektronischen Implantat verwendet, um für die jeweiligen Therapien in den einzelnen Zonen eine Therapieerfolgszählung durchzuführen. Gezählt werden dabei die Therapieerfolge der einzelnen ATP-Versuche in der VT1-Zone für stabile VT (10) und instabile VT (20). Gezählt werden die Therapieerfolge der einzelnen Schocks in der VT1-Zone, ebenfalls getrennt nach stabiler VT (30) und instabiler VT (40).

Identisch zur VT1-Zone sind in der VT2-Zone Therapieerfolgszähler für die ATP-Versuche und Schocks, getrennt nach stabiler und instabiler VT, vorgesehen.

In der VF-Zone entfällt die Trennung nach stabiler und instabiler Arrhythmie, so dass hier Therapieerfolgszähler für ATP (50) und Schocks (60) vorgesehen sind.

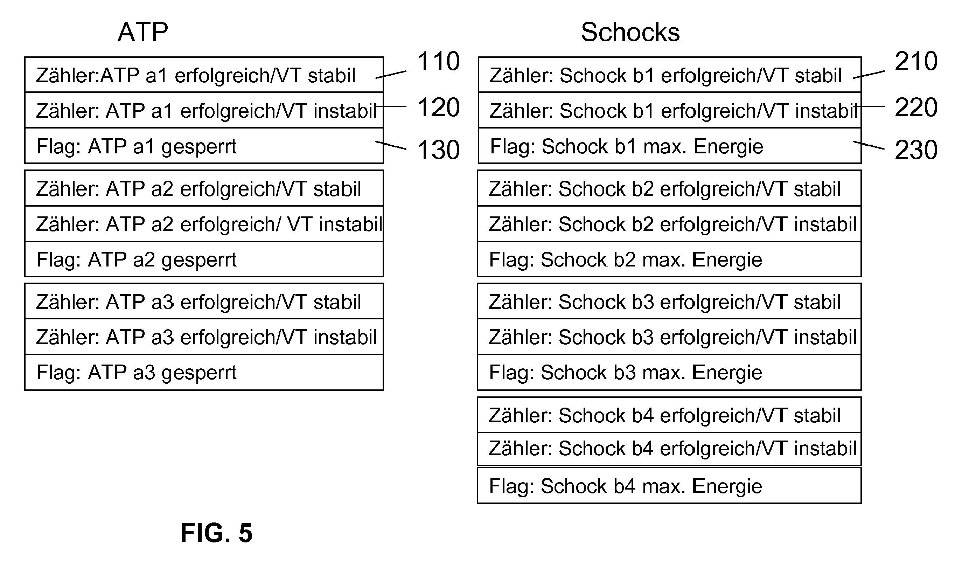

In Figur 5 ist die Struktur der Therapieerfolgszähler, getrennt für ATP und Schock, dargestellt.

Der ATP-Zähler registriert für jeden ATP-Versuch (a1, a2, a3) innerhalb einer Zone die Anzahl der erfolgreichen ATPs bei stabiler VT (110), die Anzahl der erfolgreichen ATPs bei instabiler VT (120) und verwaltet ein Flag (130), das diesen ATP-Versuch für die zukünftige Therapie sperren kann (z. B. bei Akzeleration des Rhythmus durch diesen ATP-Versuch).

Der Schock-Zähler registriert für jeden abgegebenen Schock (b1, b2, b3) innerhalb einer Zone die Anzahl der erfolgreichen Schocks bei stabiler VT (210), die Anzahl der erfolgreichen Schocks bei instabiler VT (220) und verwaltet ein Flag (230), das anzeigt, ob bei der nächsten Episode die Schockenergie des jeweiligen Schocks auf Maximalenergie vom elektronischen Implantat erhöht werden soll. So kann gesteuert werden, dass ein erfolgloser Schock mit einer Energie kleiner der Maximalenergie in Zukunft immer als Maximalenergieschock abgegeben wird und so die Patientensicherheit erhöht wird.

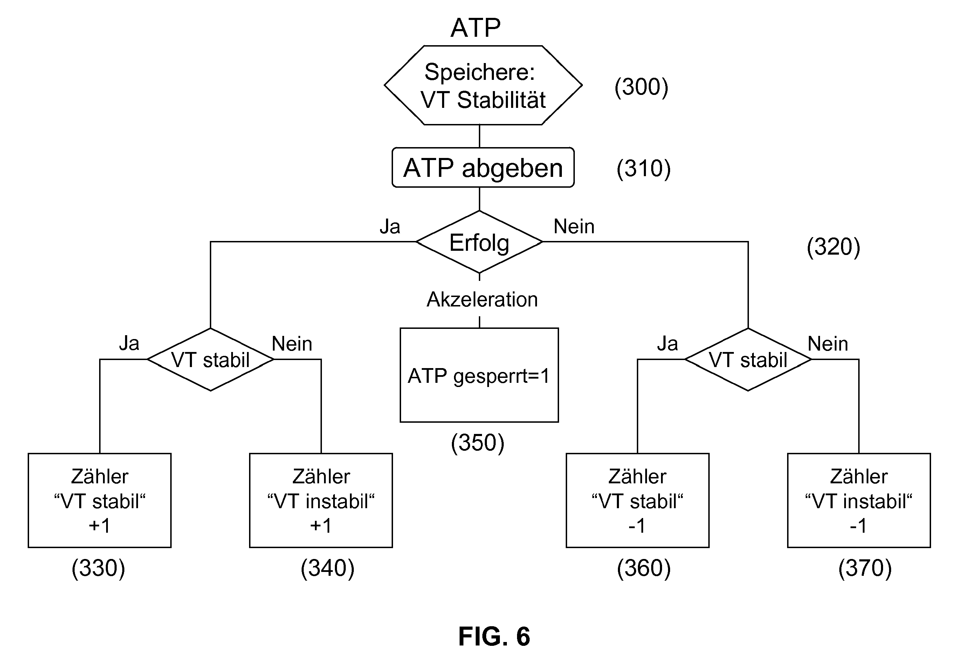

In Figur 6 ist der Ablaufplan der Therapieerfolgszählung für den ATP-Zähler in den VT-Zonen dargestellt. Unmittelbar vor der ATP-Abgabe wird im Implantat die Information über die Stabilität der zu behandelnden VT abgespeichert (300). Anschließend wird die ATP abgegeben (310) und deren Therapieerfolg in der sich anschließenden Redetektionsphase vom elektronischen Implantat bewertet (320). Erfolgt eine Terminierungsdetektion nach dieser ATP, so gilt diese als erfolgreich (Erfolg=Ja), erfolgt eine Redetektion einer VT in der selben oder einer niedrigeren VT-Zone, gilt die ATP als erfolglos (Erfolg=Nein). Erfolgt nach der ATP eine Redetektion in einer höheren VT bzw. der VF-Zone, hat die ATP die VT beschleunigt (Erfolg=Akzeleration).

Ist die ATP als erfolgreich bewertet worden, wird abhängig von Stabilitätszustand (300) der VT vor ATP-Abgabe der ATP-Erfolgszähler für eine stabile VT (330) oder eine instabile VT (340) inkrementiert.

Ist die ATP als erfolglos bewertet worden, wird abhängig von Stabilitätszustand (300) der VT vor ATP-Abgabe der ATP-Erfolgszähler für eine stabile VT (360) oder eine instabile VT (370) dekrementiert. Der minimale Zählerwert ist dabei 0.

Beschleunigt die ATP hingegen die VT in eine höhere VT bzw. in die VF-Zone, wird das Flag zum Sperren dieser ATP im Therapieerfolgszähler gesetzt (350). Die Zählerstände bleiben dabei unverändert.

In der VF-Zone entfallen die Stabilitätsbewertung und die Akzelerationsabfrage (320).

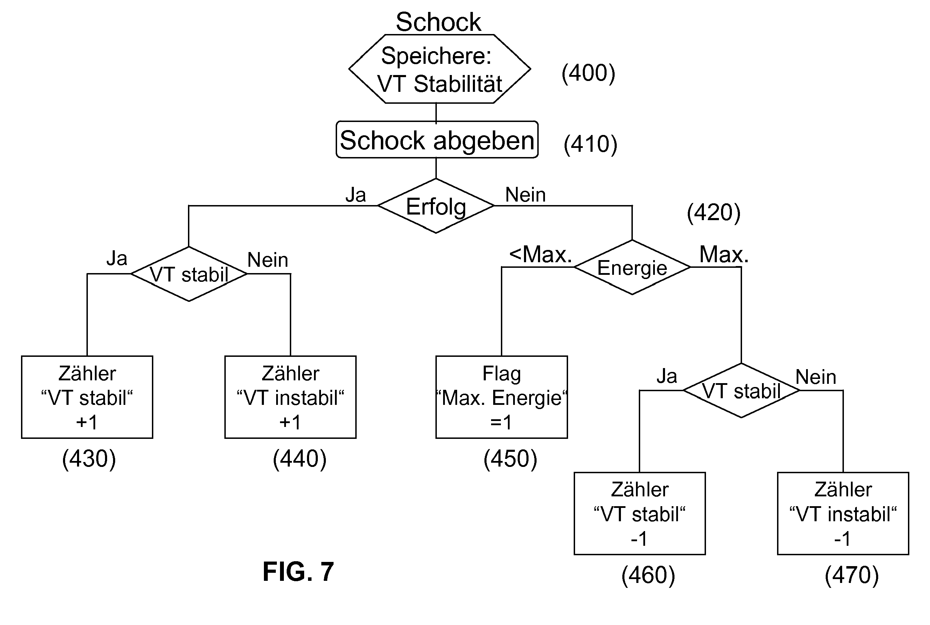

In Figur 7 ist der Ablaufplan der Therapieerfolgszählung für den Schock-Zähler in den VT-Zonen dargestellt. Unmittelbar vor dem Start des Ladevorganges für die Schocktherapie wird im Implantat die Information über die Stabilität der zu behandelnden VT abgespeichert (400). Anschließend werden die Hochspannungskondensatoren des ICD geladen und nach dessen Abschluss der Defibrillationsschock abgegeben (410) und dessen Therapieerfolg in der sich anschließenden Redetektionsphase vom elektronischen Implantat bewertet (420). Erfolgt eine Terminierungsdetektion (d. h. die Tachykardie besteht nach Schockabgabe nicht fort) nach dem Schock, so gilt dieser als erfolgreich (Erfolg=Ja), erfolgt eine Redetektion (d. h. die Tachykardie besteht nach Schockabgabe fort) einer VT oder der VF-Zone, gilt der Schock als erfolglos (Erfolg=Nein).

ist der Schock als erfolgreich bewertet worden, wird abhängig von Stabilitätszustand (400) der VT vor Ladebeginn der Schock-Erfolgszähler für eine stabile VT (430) oder eine instabile VT (440) inkrementiert.

Ist der Schock als erfolglos bewertet worden, wird zunächst geprüft, ob die Energie dieses Schocks bereits die Maximalenergie ist. Ist die Energie kleiner der maximal möglichen Schockenergie (Energie<Max.), dann wird im Schock-Zähler das Flag zur Schockenergieerhöhung auf maximale Energie gesetzt (450). Der Zählerstand wird nicht verändert.

Ist die Schockenergie bereits Maximalenergie, dann wird abhängig von Stabilitätszustand (400) der VT vor Ladebeginn der Schock-Erfolgszähler für eine stabile VT (460) oder eine instabile VT (470) dekrementiert. Der minimale Zählerwert ist dabei 0.

In der VF-Zone entfällt die Stabilitätsbewertung.

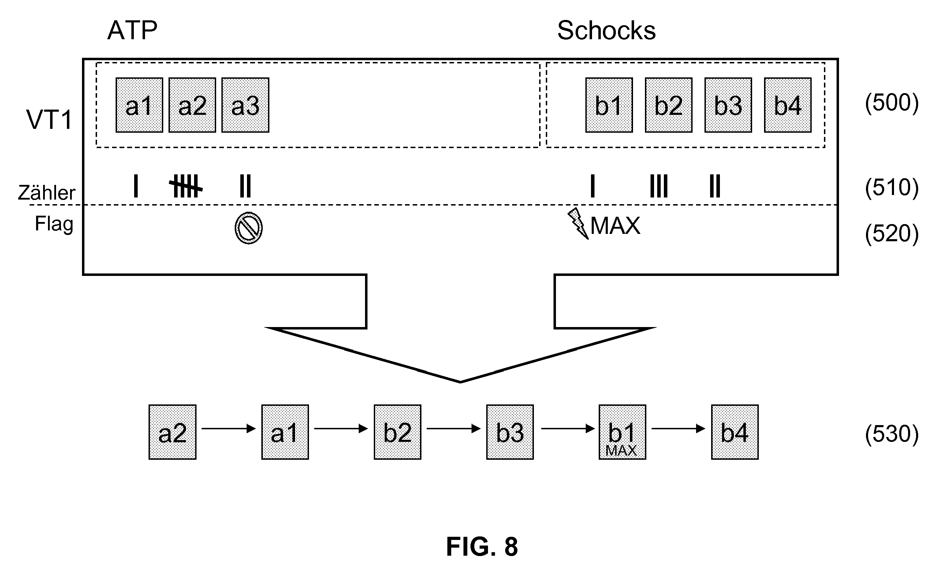

Figur 8 illustriert die automatische Umorganisation der Therapiesequenzstruktur durch die Therapieeinrichtung bzw. die Steuereinheit 54 und die Therapiesteuereinheit 92.

In der oberen Zeile ist die initial programmierte Therapiesequenz in der VT1-Zone dargestellt (500), bestehend aus 3 ATP-Versuchen (a1, a2, a3) und 4 Defibrillationsschocks (b1, b2, b3, b4), wobei im dargestellten Beispiel der erste Schock (b1) eine programmierte Energie kleiner der Maximalenergie hat.

In der nächsten Zeile sind die in der Vergangenheit aufsummierten Therapieerfolge der einzelnen Therapien illustriert (510). Die ATP a1 war 1x erfolgreich, a2 5x und a3 2x. Der erste Schock b1 war 1x effektiv, b2 3x und b3 2x.

In der folgenden Zeile sind die zusätzlichen Flags dargestellt (520). Da die ATP a3 einmal zu Akzeleration der VT geführt hat, ist das Flag zum Sperren (∅) dieser ATP gesetzt. Der erste Schock b1 war einmal ineffektiv, daher ist das Flag zur automatischen Erhöhung der Schockenergie ( 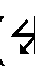 MAX) gesetzt.

Im Ergebnis der Therapieeffizienzzähler wird bei erneutem Auftreten einer VT in der VT1-Zone die Therapiesequenz entsprechend der unteren Zeile (53) abgearbeitet: Zunächst wird die erfolgreichste ATP a2, dann die ATP a1 abgegeben, gefolgt von Schock b2, dann b3, dann b1 (mit maximaler Schockenergie) und b4. Die ATP a3 wird aufgrund des gesetzten Akzelerationsflags unterdrückt.

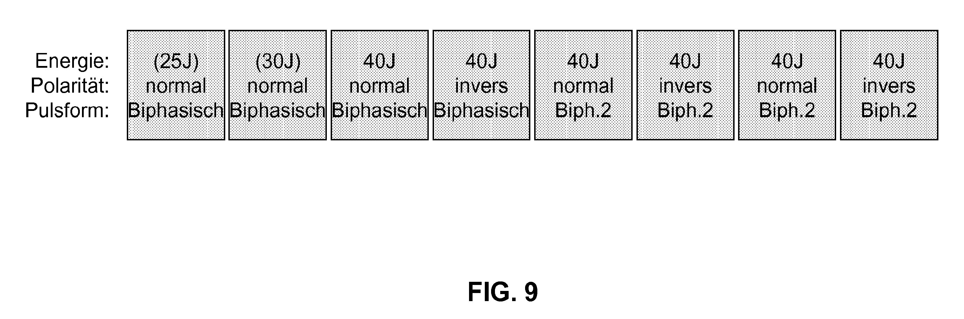

Im Folgenden sind die Anforderungen an die Implementierung einer solchen Funktion gelistet:
- Es soll einen einstellbaren Parameter "Automatische Therapieoptimierung" mit dem Wertebereich [EIN/AUS] (Standardwert AUS) geben.
- Wenn die "Automatische Therapieoptimierung" aktiviert ist, dann sollen die Parameter "Energie", "Schockpolarität" und "Schockform" für alle Schocks einer VT oder VF-Zone entsprechend Figur 9 fest eingestellt werden. Die Schockenergie der ersten beiden Schocks bleibt jedoch frei programmierbar (25J, 30J).
- Für jeden ATP-Angriff und jeden Schock in den VT-Zonen sollen zwei Therapieerfolgszähler abgespeichert werden: Therapieerfolg bei stabiler VT und Therapieerfolg bei instabiler VT.
- Für die Stabilitätsbewertung wird immer das ventrikuläre Stabilitätskriterium unmittelbar vor der jeweiligen Detektion bzw. Redetektion genutzt.
- Für jeden Schock in den VF-Zonen soll ein Therapieerfolgszähler abgespeichert werden.
- Der Therapieerfolgszähler soll inkrementiert werden, wenn die Therapie zur Terminierungsdetektion der Episode führt.
- Der Therapieerfolgszähler soll dekrementiert werden, wenn der Therapie eine Redetektion in der gleichen oder niedrigeren Zone folgt und der Zählerstand größer Null ist.
- Der Therapieerfolgszähler soll gelöscht werden (=0), wenn der Therapie eine Redetektion in einer höheren Zone (Akzeleration) folgt.
- Folgt einer ATP in einer VT-Zone eine Redetektion in der VF-Zone, dann soll im Therapieerfolgszähler ein Flag "ATP gesperrt" gesetzt werden. Der Therapieerfolgszählerstand soll in diesem Fall unverändert bleiben.
- Der Therapieerfolgszähler bleibt unverändert, wenn die Therapie (DFc) oder die Episode (Programmer) abgebrochen wurde.
- Die Therapieerfolgszähler sollen nur von spontanen Episoden beeinflusst werden, d. h. nicht bei induzierten Episoden.
- Die Therapieerfolgszähler sollen bei der Nachsorge vom Programmer abgefragt werden und dem Arzt angezeigt werden können.
- Die Therapieerfolgszähler sollen vom Arzt mit dem Programmer manuell zurückgesetzt werden können.
- Die Therapieerfolgszähler sollen bei Umprogrammierung der Therapiesequenzen, ATP und Schockparameter automatisch durch den Programmer zurückgesetzt werden.
- Wird ein Schock mit einer Energie kleiner Maximalenergie von einer Redetektion gefolgt, dann soll im Therapieerfolgszähler ein Flag "Energieerhöhung" gesetzt werden. Der Therapieerfolgszählerstand soll in diesem Fall unverändert bleiben.
- Wenn die Automatische Therapieoptimierung aktiviert ist, dann soll die Reihenfolge der Therapiesequenz entsprechend des Zählerstandes der Therapieerfolgszähler umsortiert werden:
   Zunächst wird die programmierte Anzahl der ATP-Versuche abgegeben, jedoch nicht in der programmierten Reihenfolge, sondern in der Reihenfolge absteigenden Therapieerfolges, getrennt nach stabiler und instabiler VT zum Detektions-/ Redetektionszeitpunkt.
   Sind alle ATP-Versuche erfolglos abgegeben worden, dann werden die Anzahl programmierter Schocks in der Reihenfolge absteigenden Therapieerfolges abgegeben. Ist die Energieerhöhung bei einem Schock markiert, wird die Schockenergie dieses Schocks auf Maximalenergie gesetzt.

## Patentansprüche

1. Elektrotherapieeinrichtung zur elektrischen antitachykarden Therapie eines Herzens mit
- wenigstens einer Stimulationseinheit (56, 50), die mit einer Elektrodenleitung (16) mit einer intrakardialen Stimulations- oder Defibrillationselektrode (18, 38) verbunden oder zu verbinden ist und die dazu ausgebildet ist, elektrische Stimulationsimpulse und/oder elektrische Defibrillationsschocks zu generieren und abzugeben,
- einer Steuereinheit (54), die mit der Stimulationseinheit verbunden ist und ausgebildet ist, die Stimulationseinheit zur Abgabe verschiedener antitachykarder Therapien entsprechend gespeicherter Therapiesequenzen anzusteuern, wobei eine jeweilige antitachykarde Therapie jeweils wenigstens die Abgabe eines Stimulations- oder Defibrillationsimpulses einschließt und sich verschiedene antitachykarde Therapien einer Therapiesequenz durch wenigstens ein Therapiemerkmal, wie Stärke des Stimulationsimpulses oder Defibrillationsimpulses, Anzahl der Impulse oder Reihenfolge und Zeitpunkte einzelner Impulse, voneinander unterscheiden,
- einer Tachykardieerfassungseinheit (90), die über eine Elektrodenleitung mit einer intrakardialen Wahrnehmungselektrode verbunden oder zu verbinden ist und die dazu ausgebildet ist, ein intrakardiales Elektrogrammsignal aufzunehmen und derart zu verarbeiten, dass die Tachykardieerfassungseinheit eine Tachykardie eines Vorhofs oder einer Kammer eines Herzens detektieren und eine jeweilige Tachykardie kennzeichnende Merkmale erfassen kann,
- einer Therapieerfolgserfassungseinheit (90, 92), die mit einem Therapieerfolgsspeicher (96) verbunden und ausgebildet ist, ein Ende einer tachykarden Rhythmusstörung nach Abgabe einer Therapiesequenz als Therapieerfolg zu erfassen und in dem Therapieerfolgsspeicher Daten zu speichern, die die tachykarde Rhythmusstörung charakterisieren sowie diejenige antitachykarde Therapie innerhalb einer jeweiligen Therapiesequenz, die zur erfolgreichen Beendigung der tachykarden Rhythmusstörungen geführt hat, und auf Basis der in dem Therapieerfolgsspeicher gespeicherte Daten eine Therapieerfolgsstatistik zu erstellen,
- wobei die Steuereinheit (54, 92) mit dem Therapieerfolgsspeicher (94) verbunden und dazu ausgebildet ist, auf Basis der in dem Therapieerfolgsspeicher abgelegten Therapieerfolgsstatistik die Reihenfolge von antitachykarden Therapien einer zukünftigen, nach Erfassung einer tachykarden Rhythmusstörung anzuwendenden Therapiesequenz derart zu bestimmen, dass die Reihenfolge der antitachykarden Therapien innerhalb der Therapiesequenz davon abhängt, wie häufig eine antitachykarde Therapie im Vergleich zu anderen antitachykarden Therapien der Therapiesequenz zu einer erfolgreichen Beendigung einer Tachykardie geführt hat.

2. Elektrotherapieeinrichtung nach Anspruch 1, **dadurch gekennzeichnet, dass** die Therapieerfolgsstatistik von Therapieerfolgszählerständen gebildet ist, die in dem Therapieerfolgsspeicher gespeichert sind und Zählerstände von Therapieerfolgszählern sind, die von der Therapieerfolgserfassungseinheit in Verbindung mit dem Therapieerfolgspeicher gebildet sind.

3. Elektrotherapieeinrichtung nach Anspruch 1 oder 2, **dadurch gekennzeichnet, dass** die Tachykardieerfassungseinheit ausgebildet ist, stabile und instabile Tachykardien voneinander zu unterscheiden.

4. Elektrotherapieeinrichtung nach Anspruch 3, **dadurch gekennzeichnet, dass** die Therapieerfolgserfassungseinheit in Verbindung mit dem Therapieerfolgsspeicher als Therapieerfolgszähler dazu ausgebildet ist, einen von zwei einer jeweiligen antitachykarden Therapie zugeordneten Zählerständen zu erhöhen, von denen einer instabilen Tachykardien zugeordnet ist und der andere stabilen Tachykardien, je nachdem, ob eine jeweils erfolgreich beendete Tachykardie von der Tachykardieerfassungseinheit als stabil oder als instabil klassifiziert wurde.

5. Elektrotherapieeinrichtung nach einem der Ansprüche 2 bis 4, **dadurch gekennzeichnet, dass** die Therapieerfassungseinheit ausgebildet ist, ventrikuläre Tachykardien in Abhängigkeit der Herzrate unterschiedlichen Tachykardie-Zonen zuzuordnen.

6. Elektrotherapieeinrichtung nach Anspruch 5, **dadurch gekennzeichnet, dass** für jede antitachykarde Therapie jeweils separate Therapieerfolgszähler für verschiedene Tachykardie-Zonen vorgesehen sind.

7. Elektrotherapieeinrichtung nach einem der Ansprüche 1 bis 6, **dadurch gekennzeichnet, dass** die Therapieerfassungseinheit ausgebildet ist, eine sich beschleunigende Tachykardie nach Abgabe einer antitachykarden Therapie zu erfassen.

8. Elektrotherapieeinrichtung nach Anspruch 7, **dadurch gekennzeichnet, dass** die Steuereinheit ausgebildet ist, nach Erfassen einer sich beschleunigenden Tachykardie infolge der Abgabe einer antitachykarden Therapie den zughörigen Therapieerfolgszähler um 1 herabzuzählen oder den zughörigen Therapieerfolgszählerstand auf "0" zu setzen.

9. Elektrotherapieeinrichtung nach Anspruch 7, **dadurch gekennzeichnet, dass** der Therapieerfolgsspeicher ausgebildet ist, zu einer jeweiligen antitachykarden Therapie eine Sperrkennung zu speichern, und die Therapieerfolgserfassungseinheit ausgebildet ist, die Sperrkennung zu einer jeweiligen antitachykarden Therapie in dem Therapieerfolgsspeicher zu speichern, wenn diese antitachykarde Therapie zu einer sich beschleunigenden Tachykardie geführt hat.

10. Elektrotherapieeinrichtung nach Anspruch 9, **dadurch gekennzeichnet, dass** die Steuereinheit dazu ausgebildet ist, eine mit einer Sperrkennung gekennzeichnete antitachykarde Therapie aus einer jeweils neu gebildeten Therapiesequenz ganz auszunehmen, so dass eine mit einer Sperrkennung versehene antitachykarde Therapie zur Behandlung einer jeweiligen Tachykardie, zu deren Beschleunigung die antitachykarde Therapie geführt hat, nicht mehr abgegeben wird.

11. Elektrotherapieeinrichtung nach einem der Ansprüche 2 bis 10, **dadurch gekennzeichnet, dass** die Elektrotherapieeinrichtung separate Therapieerfolgszähler für verschiedene antitachykarde Therapien in Verbindung mit verschiedenen Tachykardien aufweist.

12. Elektrotherapieeinrichtung nach einem der Ansprüche 1 bis 11, **dadurch gekennzeichnet, dass** die Therapieerfolgsstatistik mittels eines externen Programmiergerätes abgefragt werden kann.

13. Elektrotherapieeinrichtung nach einem der Ansprüche 1 bis 12, **dadurch gekennzeichnet, dass** die Therapieerfolgszählerstände in dem Therapieerfolgsspeicher einzeln oder insgesamt mittels eines externen Programmiergerätes gelöscht werden können.

14. Elektrotherapieeinrichtung nach einem der Ansprüche 1 bis 13, **dadurch gekennzeichnet, dass** die Steuereinheit ausgebildet ist, die Therapieerfolgszählerstände in dem Therapieerfolgsspeicher bei einer Umprogrammierung der Therapiesequenz automatisch anzupassen, insbesondere zu löschen oder zu korrigieren.

15. Elektrotherapieeinrichtung nach einem der Ansprüche 1 bis 14, **dadurch gekennzeichnet, dass** die Therapiesequenzoptimierung durch die Steuereinheit mittels eines programmierbaren Parameters aktivier- oder deaktivierbar ist.
